# EUROPÄISCHE PATENTSCHRIFT

(11) **EP 1 265 633 B1**
(45) Veröffentlichungstag und Bekanntmachung des Hinweises auf die Patenterteilung: **12.12.2012**
(21) Anmeldenummer: 01931522.5
(22) Anmeldetag: 14.03.2001
(51) Int. Cl.: A61K 39/39, A61K 39/295, A61K 39/116, A61P 37/04, A61P 31/00

(54) **ADJUVANS FÜR VAKZINEN**
ADJUVANT FOR VACCINES
ADJUVANT POUR VACCINS

(30) Priorität: 14.03.2000 DE 10012370
(43) Veröffentlichungstag der Anmeldung: 18.12.2002
(73) Patentinhaber: Novartis Vaccines and Diagnostics GmbH, 35041 Marburg (DE)
(72) Erfinder: BROKER, Michael, 35041 Marburg (DE)
(74) Vertreter: Marshall, Cameron John
(86) Internationale Anmeldenummer: PCT/EP2001/002866
(87) Internationale Veröffentlichungsnummer: WO 2001/068129

(56) Entgegenhaltungen:
- EP-A2- 1 951 302
- WO-A-96/21465
- S. DE DONATO ET AL.: "Safety and immunogenicity of MF59-adjuvanted influenza vaccine in the elderly." VACCINE, Bd. 17, 1999, Seiten 3094-3101, XP002181050 GUILDFORD, GB in der Anmeldung erwähnt
- D.M. GRANOFF ET AL.: "MF59 adjuvant enhances antibody responses of infant baboons immunized with Haemophilus influenzae type b and Neisseria meningitidis group C oligosaccharide-CRM 197 conjugate vaccine." INFECTION AND IMMUNITY, Bd. 65, Nr. 5, Mai 1997 (1997-05), Seiten 1710-1715, XP002181051 WASHINGTON, US
- M. DUPUIS ET AL.: "Distribution of adjuvant MF59 and antigen gD2 after intramuscular injection in mice." VACCINE, Bd. 18, Nr. 5-6, 14. Oktober 1999 (1999-10-14), Seiten 434-439, XP002181052 GUILDFORD, GB in der Anmeldung erwähnt
- SANKILAMPI U ET AL: "Associations of prevaccination antibody levels with adverse reactions to pneumococcal and influenza vaccines administered simultaneously in the elderly", VACCINE, BUTTERWORTH SCIENTIFIC. GUILDFORD, GB, vol. 15, no. 10, 1 July 1997 (1997-07-01), pages 1133-1137, XP004085974, ISSN: 0264-410X
- O'HAGAN D T ET AL: "The path to a successful vaccine adjuvant - 'The long and winding road'", DRUG DISCOVERY TODAY, ELSEVIER, RAHWAY, NJ, US, vol. 14, no. 11-12, 1 June 2009 (2009-06-01), pages 541-551, XP026158111, ISSN: 1359-6446 [retrieved on 2009-03-05]
- OTT G ET AL: "DESIGN AND EVALUATION OF A SAFE AND POTENT ADJUVANT FOR HUMAN VACCINES", VACCINE DESIGN: THE SUBUNIT AND ADJUVANT APPROACH, XX, XX, 1 January 1995 (1995-01-01), pages 277-296, XP001248048,
- DUPUIS M ET AL: "Distribution of adjuvant MF59 and antigen gD2 after intramuscular injection in mice", VACCINE, ELSEVIER LTD, GB, vol. 18, no. 5-6, 14 October 1999 (1999-10-14), pages 434-439, XP002181052, ISSN: 0264-410X, DOI: 10.1016/S0264-410X(99)00263-7

## Beschreibung

Die Erfindung betrifft die Verwendung einer Öl-in-Wasser-Emulsion als kontralateral zu applizierendes Adjuvans. Im besonderen betrifft die Erfindung Vakzinen enthaltend eine mit einer Öl-in-Wasser-Emulsion adjuvierte erste Vakzine und eine nicht mit diesem Adjuvans adjuvierte zweite Vakzine als Kombinationspartner zur gleichzeitigen, getrennten oder zeitlich abgestuften Anwendung zur Therapie oder Prophylaxe. Im ganz besonderen betrifft die Erfindung Kombinationen aus einer mit MF59 adjuvierten Influenzavakzine und einer zweiten Vakzine.

Zahlreiche Impfstofformulierungen, die attenuierte Pathogene oder Protein-Subunit-Antigene enthalten, sind bisher entwickelt worden. Konventionelle Impfstoffzubereitungen enthalten zumeist Adjuvantien zur Verstärkung der Immunantwort. Zum Beispiel werden häufig Depot-bildende Adjuvantien verwendet, welche das verabreichte Antigen absorbieren und/oder präzipitieren und ein Depot an der Injektionsstelle ausbilden. Typische Depot-bildende Adjuvantien schließen Aluminiumverbindungen (Alum) und Wasser-in-Öl-Emulsionen ein. Jedoch rufen Depot-bildende Adjuvantien, obwohl sie die Antigenität erhöhen, häufig schwere andauernde lokale Reaktionen, wie Granulome, Abszesse und Narben hervor, wenn sie subcutan oder intramuskulär appliziert werden.

Andere Adjuvantien, wie Lipopolysaccharide und Muramyldipeptide können bei Injektion pyrogene Reaktionen oder das Reitersyndrom, wie etwa Grippe-ähnliche Symptome, generalisierter Gelenkschmerz und manchmal auch Uveitis anterior, Arthritis und Urethritis hervorrufen. Saponine, wie aus *Quillaja saponaria,* sind ebenfalls als Adjuvantien in Impfstoffen verwendet worden.

Vor kurzem ist MF59, eine in ihrer Anwendung sichere immunstimulierende Submikro-Öl-in-Wasser-Emulsion zur Verwendung in Impfstofformulierungen entwickelt worden. Siehe z.B., Ott et al., "MF59-Design and Evaluation of a Safe and Potent Adjuvant for Human Vaccines" in Vaccine Design: The Subunit and Adjuvant Approach (Powell, M.F. and Newman, M.J. Herausgeber) Plenum Press, New York, 1995, Seiten 277-296. Bis heute sind nur Aluminiumsalze und MF59 zur Verwendung als Adjuvantien zur Impfstofformulierung zur Applikation am Menschen zugelassen.

Adjuvantien können auf verschiedene Arten wirken, sie können das Cytokin-Netzwerk beeinflussen, Antigene zu potenten Antigen-präsentierenden Zellen dirigieren, cytotoxische T-Lymphozyten induzieren oder sie können die Freisetzung des Antigens durch Depotbildung verlängern. Die konventionelle Anwendung von Adjuvantien und Vakzine ist üblicherweise zeit- und ortsgleich, um eine Immunantwort gegen das applizierte Antigen zu erhöhen.

Für MF59 wurde eine zeitliche und räumliche Trennung der Applikation von Antigen und Adjuvans im Tierversuch allerdings ohne genauere Angaben zu den unterschiedlichen Applikationssorten beschrieben (Dupuis et al., Vaccine 18 (2000), 434-439, Dupuis et al., Cellular Immunology 186 (1998), 18-27 und Ott et al., "MF59-Design and Evaluation of a Safe and Potent Adjuvant for Human Vaccines" in Vaccine Design: The Subunit and Adjuvant Approach (Powell, M.F. and Newman M.J. Herausgeber) Plenum Press, New York, 1995, Seiten 277-296), welche dennoch zu einer Steigerung der applizierten Immunität/Antigenität der zeitlich bzw. räumlich getrennten Antigene führte. Jedoch ist eine (simultan) kontralaterale Applikation von MF59 bzw. eine mit MF59 adjuvierten Vakzine in Kombination mit einer zweiten, nicht mit MF59 adjuvierten Vakzine noch nirgendwo beschrieben worden.

Sankilampi et al. (Vaccine. 1997 Jul;15(10):1133-7) beschreibt eine Studie in der älteren Patienten simultan ein Pneumokokken und ein Grippeimpfstoff verabreicht wurden.

Die vorliegende Erfindung basiert auf der überraschenden und unerwarteten Entdeckung, daß die räumlich getrennte konsekutive oder simultane Applikation von MF59 oder eines mit MF59 adjuvierten Impfstoffs einen synergistischen Effekt auf die Antigenität/Immunogenität eines zweiten, nicht mit MF59 adjuvierten Impfstoffs beim Menschen erzielt.

Dieser Effekt ist aufgrund der in der Literatur diskutierten Wirkungsweise von MF59 nicht zu erwarten. Hierbei ist zu beachten, daß der Wirkmechanismus für MF59 noch nicht gänzlich aufgeklärt ist.

Zwar wird eine Stimulierung der Cytokinsynthese v.a. von IL-5 und IL-6 diskutiert (z.B. Cellular Immunology, 186 (1998), Seiten 18-27), jedoch hat sich insbesondere gezeigt, daß MF59 die Rekrutierung und Aktivierung von antigen-präsentierenden Zellen wie dendritischer Zellen z.B. im Muskel bewirkt, welche das Antigen aufnehmen und in die ableitenden Lymphknoten (draining lymph nodes) wandern und das prozessierte Antigen den T-Lymphocyten effektiv präsentieren, was zumindest als Hinweis zu deuten ist, daß eine gewisse räumliche Nähe der Applikationsstelle von Adjuvans oder Antigen im Muskel gegeben sein sollte. Obwohl, wie oben ausgeführt, ein Tierversuch die räumlich getrennte Applikation von MF59 und Antigen zu einer Adjuvierung (Stimulierung der Antigenität/Immunogenität) führte, sind die gefundenen Effekte bei der kontralateralen Applikation beim Menschen umso erstaunlicher, wenn man bedenkt, daß es, wie dem Fachmann hinlänglich bekannt, nicht möglich ist, insbesondere die mit Adjuvantien im Tierversuch mit Kleinsäugern erhaltenen Ergebnisse auf große Säuger - vom Menschen ganz zu schweigen - zu extrapolieren. Dies ist vor allem bei der kontralateralen Applikation zu beachten, da bei Kleinsäugern natürlich die räumliche Trennung nicht so deutlich ausfällt.

Die kontralateral simultane Applikation der beiden Vakzinen, von welche eine mit MF59 adjuviert ist und einer zweiten Vakzine, die nicht mit MF59 adjuviert ist, die bevorzugte Ausführungsform der vorliegenden Erfindung. "Kontralateral", wie in der vorliegenden Beschreibung und den Ansprüchen verwendet, bezeichnet die Applikation an entgegengesetzten Körperseiten, wie z.B. üblicherweise in den Deltamuskel (Musculus deltoides) des rechten und des linken Oberarms.

Die Applikation kann konsekutiv oder simultan erfolgen, wobei die simultane Applikation bevorzugt ist.

Die bevorzugt als Adjuvans verwendete Öl-in-Wasser-Emulsion ist MF59, deren Zusammensetzung und Herstellung nachfolgend beschrieben ist:
1. Squalen (2, 6, 10, 15, 23-Hexamethyl-2, 6, 10, 14, 18, 22-Tetracosahexan) ca. 5% (39 mg/ml)
2. Polysorbat 80 (Tween ® 80) ca. 0,5% (4,7 mg/ml)
3. Sorbitantrioleat 85 (Span® 85) ca. 0,5% (4,7 mg/ml)
4. Citratpuffer pH 6,5 (Trinatriumcitratdihydrat, Citronensäuremonohydrat, Wasser zur Injektion)

Die Herstellung von MF59 erfolgt in an sich bekannter Weise (Ott et al., "MF59-Design and Evaluation of a Safe and Potent Adjuvant for Human Vaccines" in Vaccine Design: The Subunit and Adjuvant Approach (Powell, M.F. and Newman M.J. Herausgeber) Plenum Press, New York, 1995, Seiten 277-296)).

Polysorbat 80 wird in Wasser zur Injektion gelöst und mit Natriumcitratpuffer versetzt. Separat wird Sorbitantrioleat in Squalen gelöst. Diese beiden Lösungen werden vereinigt und in einem Homogenisator (Mikrofluidizer) wird eine Emulsion hergestellt. Nach der Filtration durch einen 22µm-Filter und Entfernung größerer Tropfen unter Stickstoffbehandlung entsteht eine milchige, weiße stabile Emulsion, welche im wesentlichen Partikel mit einem Durchmesser <1,2 µm enthält. Die entstandene Emulsion kann der zu adjuvierenden Vakzine entweder bei Herstellung oder auch erst kurz vor der Applikation zugemischt werden, wie z.B. bei Formulierung mit dem rekombinant hergestellten Oberflächenglykoprotein gp12O des Humanen Immunodefizienz Virus (HIV), um Konfirmationsänderungen zu verhindern. Auch ist eine proximale Applikation von Antigen und MF59 möglich.

"Vakzine", wie in der Beschreibung und den Ansprüchen verwendet, bezeichnet virale, bakterielle oder parasitäre Antigene. Diese können in Form von ganzen (whole-cell) Viren, Bakterien, Parasiten, Protein-Subunits, Polysacchariden, Polysaccharidkonjugaten und Nucleinsäuren vorliegen. Sie können galenisch unverändert oder auch in Zusammenhang mit Vehikeln oder Trägern wie z.B. Microsphären, Liposomen, Nanosphären, ISCOMS und weiteren dem Fachmann bekannten "antigen delivery"-Systemen verwendet werden.

Wie bereits obenstehend erwähnt, ist eine besonders bevorzugte Ausführungsform der Erfindung die kombinierte simultan kontralaterale Applikation einer mit MF59 adjuvierten Influenza-Protein-Subunit-Vakzine, wie Fluad® mit einer nicht adjuvierten Kapselpolysaccharid-Vakzine gegen Streptococcus pneumoniae. Die kontralateral simultane Applikation dieser beiden Vakzinen bietet sich insbesondere deswegen an, weil der Personenkreis für den die Impfung mit beiden Vakzinen empfohlen ist, sich weitgehend deckt. Eine Grippeimpfung wurde von der Ständigen Impfkommission des Robert-Koch-Instituts (STIKO) besonders bei immungeschwächten Patienten (z.B. Immunsupression durch hochdozierte Steroidbehandlung, Zustand nach Transplantationen, Dialyse-Patienten), speziellen Risikogruppen, wie Diabetikern und Bewohnern von Seniorenheimen empfohlen. Genau dieser Personenkreis ist nicht nur durch eine Influenzainfektion besonders gefährdet, sondern hat auch ein erhöhtes Risiko durch PneumokokkenInfektion. Bakterien der Spezies Streptococcus pneumoniae sind die häufigsten Erreger der eitrigen Bronchitis und der bakteriellen Lungenentzündung. Weitere schwere Pneumokokkenerkrankungen sind die akute eitrige Meningitis, die akute Endokarditis, Sepsis und Peritonitis. Pneumokokken-Pneumonien weisen eine Letalitätsrate von 10% auf und Risikofaktoren, wie sie in der oben genannten Personengruppe zu finden sind, erhöhen die Letalität bis auf 20-30%. Ab dem 50. Lebensjahr ist die Letalität sogar noch höher.

Die Virusgrippe oder Influenza ist eine beim Menschen akut fieberhaft verlaufende Infektionskrankheit, die gewöhnlich epidemisch gehäuft auftritt und sich rasch über Kontinente als Pandemie verbreiten kann. Die Infektion mit Influenzaviren tritt vorzugsweise in den Wintermonaten auf. Es sind drei unterschiedliche Influenzavirustypen bekannt: Influenzavirus A, B und C. Influenzaviren sind RNA-Viren und werden der Familie Orthomyxoviren zugeordnet. Das Influenzavirus ist komplex aufgebaut. Es besteht aus einem fadenförmigen Ribonukleokapsid, welches von einer Hülle umgeben ist. Auf der Außenseite der Hülle sind die Antigene Hämagglutinin (HA) und Neuraminidase (NA) integriert. Diese beiden Antigene sitzen wie pilzförmige Aufsätze (spikes) auf der Partikeloberfläche. HA und NA sind für die Adhäsion und intrazelluläre Penetration des Virus von Bedeutung. Beim Influenzavirus, das den Menschen infizieren kann, sind drei HA-Serotypen (H1, H2 und H3) und zwei NA-Typen (NA1 und NA2) bekannt. Umfangreiche präklinische und klinische Studien haben gezeigt, daß das HA schützende, Virus-neutralisierende Antikörper zu induzieren vermag.

Das Influenzavirus zeichnet sich durch eine genetische Besonderheit aus: Die virale Ribonukleinsäure (RNA) ist in acht Segmente unterteilt, die einzeln an die Virusnachkommen weitergegeben werden können. Damit besteht die Möglichkeit einer beliebigen Neukombination zwischen Viruspartikeln eines Virustyps. Der Virustyp A unterliegt dem Phänomen des Antigenwandels durch Antigendrift und Antigenshift. Als Antigendrift wird eine Punktmutation im HA-Gen bezeichnet. Neue Driftvarianten sind verantwortlich für das Auftreten von Epidemien. Unter Antigenshift versteht man den Austausch von größeren Genabschnitten zwischen verschieden tierischen und humanen Influenzastämmen (Reassortment der RNA-Segmente). So wechselten 1957 die Oberflächenantigene H1 N1 durch Austausch von homologen RNA-Segmenten zwischen humanen und tierischen Influenzavirusstämmen in H2N2 und 1968 H2N2 in H3N2. Die Virusgrippe ist eine hochkontagiöse, weltweit vorkommende Erkrankung, die typischerweise pandemisch durch den Typ A, epidemisch durch den Typ B und nur sporadisch durch den Typ C hervorgerufen wird.

Epidemien mit Influenza A und B führen insbesondere im Vorschul- und Schulalter zu hohen Infektionsraten. Erwachsene die in Gemeinschaft mit Kleinkindern leben, unterliegen einem besonders hohen Risiko zu erkranken. Erkrankungen durch Influenzavirus A verlaufen moderat bis schwerwiegend und betreffen alle Bevölkerungsgruppen. Besonders gefährdet sind Personen mit chronischen Erkrankungen des Herz-Kreislauf-Systems, der Atemwege, mit Stoffwechselstörungen, Immunfunktionsstörungen und Nierenerkrankungen. Auch Menschen mit angeborenen Herzfehlern besitzen ein besonders hohes Risiko nach einer Infektion mit Influenzaviren.

Zur Prävention stehen wirksame Vakzinen zur Verfügung. Es werden drei verschiedene Impfstofftypen angeboten: inaktivierter Vollpartikel-, Spalt und Subunit-Impfstoff. In Deutschland werden zur Zeit nur Spalt- und Subunit-Vakzinen angeboten. Diese Influenzavakzinen enthalten hochgereinigte, gespaltene und inaktivierte Viruspartikel, wobei die Subunitvakzinen nur die Virus-spezifischen Oberflächenantigene HA und NA enthalten und die Spaltvakzinen darüberhinaus auch noch virale Matrixproteine. Die Impfstoffe enthalten die Antigene je eines Vertreters der Influenzavirustypen, die jährlich von der WHO für den aktuellen Impfstoff der jeweiligen Saison festgelegt wird. Zur Zeit sind dies je ein Influenzavirus A Stamm der Formel H3N2 und H1N1 sowie ein Stamm von Influenzavirus B.

Laut einer "Note for Guidance on Harmonization fo Requirements for Influenza Vaccines" des "Committee for Proprietary Medicinal Products" der "European Agency for Evaluation of Medicinal Products" sind die Mindestanforderungen hinsichtlich der Zusammensetzung und der Potenz der Influenzavakzinen vereinheitlicht worden und alle Influenzavakzinen enthalten z.B. je 15 µm HA eines jeden der drei Stämme pro Impfdosis.

Die Wirksamkeit einer Grippeimpfung vor Erkrankung liegt bei gesunden Erwachsenen bei über 75%. Bei älteren Menschen über 60 Jahren und Immungeschwächten liegt die Schutzrate wesentlich niedriger. Allein in Deutschland versterben an einer Influenzagrippe nach Schätzungen der "Arbeitsgemeinschaft Influenza" ca. 5.000 bis 10.000 Menschen, vorwiegend Menschen aus den Risikogruppen.

Um die protektive Wirkung der Influenzavakzinen besonders in den Risikogruppen zu erhöhen, wurden zahlreiche Versuche unternommen, dies durch Zusatz von Adjuvantien zu erreichen. Eines der gängigsten Adjuvantien für Humanvakzinen sind Aluminiumsalze wie Aluminiumhydroxid (Alum) und Aluminiumphosphat. Alum ist Bestandteil zahlreicher inaktivierter oder Subunitvakzinen, u.a. bei Tetanus, Diphtherie, Pertussis und Hepatitis B Virus-Impfstoffen. Bei Influenzavirus-Vakzinen ist in Tierversuchen nachgewiesen worden, daß die adjuvierten Antigene in Spalt- oder Subunitvakzinen den entsprechenden Fluid-Vakzinen überlegen sind. Daraufhin wurde auch ein Human-Spaltimpfstoff entwickelt, der mit Alum adjuviert wurde. In klinischen Studien zeigte sich aber kein statistisch signifikanter Unterschied in der Serokonversionsrate gegenüber dem adjuvansfreien Influenza-Fluidimpfstoff (Lehmann, Die gelben Hefte, 21, 76-80 (1981)). Darüber hinaus zeigte der adjuvierte Influenzaimpfstoff erhöhte lokale Impfreaktion, so daß die Adjuvierung von Influenzaimpfstoffen generell nicht zu empfehlen ist und in der Tat ist bis heute kein mit Alum adjuvierter humaner Influenzaimpfstoff auf dem Markt.

In klinischen Versuchen wurden die Immunogenität und Verträglichkeit von einem Influenza-Subunitimpfstoff (Agrippal®) und Agrippal adjuviert mit MF59 (Fluad®) vergleichend getestet. Es zeigte sich, daß der adjuvierte Impfstoff sicher und gut verträglich ist und der Zusatz von MF59 in der Vakzine erhöhte die Immunogenität der Influenzavakzine insbesondere bei Älteren mit geringen prävakzinalen Titem (De Donata et al. Vaccine 17, 3094-3101 (1999)). Auch im Vergleich zu einer nichtadjuvierten Splitvakzine zeigte sich die Überlegenheit von Fluad® (Menegon et al. Eur. J. Epidemiol. 15, 573-576 (1999)).

Fluad® wurde 1997 in Italien zugelassen und ist seit der Grippesaison 1997/1998 kommerziell in Italien erhältlich. Aufgrund des Wirkprofils bietet sich Fluad® insbesondere bei folgenden Personen an:
- Immungeschwächte Patienten
   (z.B. Immunsuppression durch hochdosierte Steroidbehandlung, Zustand nach Transplantation, Dialysepatienten)
- Spezielle Risikogruppen wie Diabetiker
- Bewohner von Seniorenheimen.

Wie bereits vorstehend erwähnt, deckt sich dieser Personenkreis weitgehend mit dem, für den ein erhöhtes Risiko durch eine Pneumokokkeninfektion besteht. Die zur Zeit verfügbaren Pneumokokken-Impfstoffe bestehen vor allem aus gereinigten Kapselpolysacchariden der 23 wichtigsten Serotypen von S. pneumoniae, z.B. die in Deutschland verfügbaren Pneumokokken-Impfstoffe Pneumopur® und Pneumovax 23® konnten in den meisten randomisierten, kontrollierten klinischen Studien keine protektive Wirksamkeit gegen Pneumokokkenpneumonie belegen. Trotzdem wird die Pneumokokkenimpfung in den Industrieländern von den jeweiligen nationalen Ministerien, Ärzteverbänden und beratenden Gremien empfohlen u.a. auch für ältere Personen, immungeschwächte Erwachsene und auch Kinder mit chronischen Erkrankungen (Impfempfehlungen der STIKO). Bestrebungen, die jetzt verfügbaren Pneumokokkenvakzinen zu optimieren, sind weithin fehlgeschlagen, da die Antigenenmenge aus Polysaccharid und Proteinträger der Vakzine enorm ansteigen würde und die Verträglichkeit unbefriedigend wäre. Außerdem wäre die Vakzine aufgrund der eingesetzten Proteinkonjugat-Technologie in der Herstellung wesentlich teurer als eine reine Polysaccharidvakzine. Der direkte Zusatz des Adjuvans MF59 zur Vakzinepräparation hingegen könnte unvorhergesehene negative Effekte zur Folge haben und aufwendige Untersuchungen zur Physikochemie der Antigene, der Immunität und der Verträglichkeit haben.

Dies gilt, wie für den Fachmann selbstverständlich, natürlich auch für den Zusatz von MF59 zu jeder anderen Vakzinepräparation. Eine überraschend gefundene Alternative zur Optimierung der Wirksamkeit schon bestehender Vakzinen, im besonderen von Pneumokokken-Polysaccharidvakzinen oder Pneumokokken-Polysaccharidkonjugat-Vakzinen besteht nun darin, diese simultan zu einer mit einer MF59 adjuvierten Influenzavakzine kontralateral zu applizieren. Das in der mit MF59 adjuvierten Vakzine enthaltene Adjuvans erhöht überraschenderweise nicht nur die Immunogenität des Influenzavirus-spezifischen Antigens, sondern auch die Immunogenität der Pneumokokken-Polysaccharid-Antigene bzw. Polysaccharid-Konjugat-Antigene. Zudem bleibt der durch die Vakzine induzierte Schutztiter länger auf einem höheren Niveau, so daß eine erneute Pneumokokkenimpfung erst in einem größeren Abstand durchgeführt werden muß.

Weitere bevorzugte Ausführungsformen der vorliegenden Erfindung ist die Verwendung von MF59 adjuvierten Proteinsubunit-Influenza-Impfstoffen in Kombination mit einer Rabiesvakzine zur postexpositionellen Prophylaxe von Tollwut, die simultan kontralaterale Applikation mit Tetanus- oder Diphtherie-Vakzinen, z.B. bei durch Hämodialyse geschwächten Patienten, die simultan kontralaterale Applikation mit dem HBV-Oberflächenantigen oder HIV-Antigenen wie gp120, die simultan kontralaterale Applikation mit einer Vakzine gegen das Frühsommer-Meningoencephalitis Virus (FSME) und die simultan kontralaterale Applikation mit weiteren Polysaccharidimpfstoffen wie z.B. gegen Typhus und Meningokokken A und/oder C, sowie weiteren Meningokokkenserotypen.

## Patentansprüche

1. Öl-in-Wasser-Emulsion die 5% Squalen, 0,5% Polysorbat 80 und 0,5% Sorbitantrioleat in wässrigem Citratpuffer pH 6,5 umfasst, zur Verwendung als kontralaterale Adjuvans für eine zweite Vakzine, wobei die besagte Emulsion und die zweite Vakzine an entgegengesetzten Körperseiten des Menschen zu applizieren sind, und wobei die zweite Vakzine und eine erste Vakzine an entgegengesetzten Körperseiten des Menschen zu applizieren sind,
wobei
(i) die erste Vakzine dadurch adjuviert wird, dass die besagte Emulsion der zu adjuvierenden ersten Vakzine entweder bei Herstellung der Vakzine oder auch erst kurz vor der Applikation zugemischt wird, und wobei
(ii) die zweite Vakzine nicht dadurch adjuviert wird, dass die besagte Emulsion der zweiten Vakzine entweder bei Herstellung der Vakzine oder auch erst kurz vor der Applikation zugemischt wird.

2. Öl-in-Wasser-Emulsion zur Verwendung nach Anspruch 1 zur Immunisierung gegen virale, bakterielle oder parasitäre Infektionskrankheiten.

3. Öl-in-Wasser-Emulsion zur Verwendung nach Anspruch 2, wobei die erste Vakzine und die zweite Vakzine simultan kontralateral an gegenüberliegenden Körperseiten des Menschen zu applizieren sind.

4. Öl-in-Wasser-Emulsion zur Verwendung nach einem der Ansprüche 1 bis 3, wobei die erste adjuvierte Vakzine eine Influenza- Proteinsubunit-Vakzine ist.

5. Öl-in-Wasser-Emulsion zur Verwendung nach Anspruch 4, wobei die erste adjuvierte Vakzine eine Influenza-Proteinsubunit-Vakzine ist und die zweite nicht-adjuvierte Vakzine eine Pneumokokken-Kapselpolysaccharid-Vakzine ist oder eine Pneumokokken-Polysaccharid-Konjugat-Vakzine ist.

6. Öl-in-Wasser-Emulsion zur Verwendung nach Anspruch 5, wobei die Adjuvans und Vakzinen zur Immunisierung gegen Influenza und Pneumokokkeninfektionen zu applizieren sind.

7. Öl-in-Wasser-Emulsion zur Verwendung nach einem der Ansprüche 1 bis 6, wobei die zweite Vakzine mit einer Aluminiumverbindung adjuviert ist.

8. Öl-in-Wasser-Emulsion zur Verwendung nach einem der Ansprüche 1-7, wobei die zweite Vakzine eine Nukleinsäure-Vakzine ist.

9. Öl-in-Wasser-Emulsion zur Verwendung nach einem der Ansprüche 1-7, wobei die zweite Vakzine eine whole-cell-Vakzine ist.

10. Öl-in-Wasser-Emulsion zur Verwendung nach einem der Ansprüche 1-7, wobei die zweite Vakzine eine Protein-Subunit-Vakzine ist.

11. Öl-in-Wasser-Emulsion zur Verwendung nach einem der Ansprüche 1-7, wobei die zweite Vakzine eine Polysaccharid-Vakzine ist.

12. Öl-in-Wasser-Emulsion zur Verwendung nach einem der Ansprüche 1-7, wobei die zweite Vakzine eine Polysaccharid-Konjugat-Vakzine ist.

13. Öl-in-Wasser-Emulsion zur Verwendung nach einem der Ansprüche 1-12, wobei die zweite Vakzine therapeutisch und/oder postexpositionell eingesetzt wird.

14. Öl-in-Wasser-Emulsion zur Verwendung nach einem der Ansprüche 1-4 und 7-13, wobei die zweite Vakzine eine Rabies-Vakzine ist.

15. Öl-in-Wesser-Emulsion zur Verwendung nach Anspruch 14 wobei die Adjuvans und die Vakzine zur postexpositionellen Tollwutprophylaxe zu applizieren sind.

16. Öl-in-Wasser-Emulsion zur Verwendung nach einem der Ansprüche 1-6 oder 11-13, wobei die zweite Vakzine eine Pneumokokken-Kapselpolysaccharid-Vakzine ist oder eine Pneumokokken-Polysaccharid-Konjugat-Vakzine ist.

17. Öl-in-Wasser-Emulsion zur Verwendung nach einem der Ansprüche 1-4 oder 7-13, wobei die zweite Vakzine eine Diphterie-Vakzine, eine Tetanus-Vakzine, eine Meningokokken-Vakzine gegen Serotyp A, C oder weitere Serotypen, eine HIV-Vakzine, eine HBV-Vakzine, eine Helicobacter-pylori-Vakzine, eine Frühsommer-Meningoencephalitis-Vakzine (FSME) oder eine Typhus-Vakzine ist.

18. Öl-in-Wasser-Emulsion zur Verwendung nach einem der Ansprüche 1 bis 18, wobei die Adjuvans und die Vakzinen bei immungeschwächten Patienten, Diabetikern oder Bewohnern von Seniorenheimen anzuwenden sind

## Claims

1. Oil-in-water emulsion which comprises 5% squalene, 0.5% polysorbate 80 and 0.5% sorbitan trioleate in an aqueous citrate butter pH 6.5, for use as contralateral adjuvant for a second vaccine, where said emulsion and the second vaccine are to be applied to opposite sides of the human body, and where the second vaccine and a first vaccine are to be applied to opposite sides of the human body, where
(i) the first vaccine is adjuvanted by mixing in said emulsion of the first vaccine to be adjuvanted either during production of the vaccine or else not until shortly prior to application, and where
(ii) the second vaccine is not adjuvanted by mixing in said emulsion of the second vaccine either during production of the vaccine or else not until shortly prior to application.

2. Oil-in-water emulsion for the use according to Claim 1 for immunization against viral, bacterial or parasitic infectious diseases.

3. Oil-in-water emulsion for the use according to Claim 2, where the first vaccine and the second vaccine are to be applied simultaneously contra-laterally to opposite sides of the human body.

4. Oil-in-water emulsion for the use according to one of Claims 1 to 3, where the first adjuvanted vaccine is an influenza-protein subunit vaccine.

5. Oil-in-water emulsion for the use according to Claim 4, where the first adjuvanted vaccine is an influenza-protein subunit vaccine and the second non-adjuvanted vaccine is a pneumococcal capsular polysaccharide vaccine or a pneumococcal polysaccharide conjugate vaccine.

6. Oil-in-water emulsion for the use according to Claim 5, where the adjuvant and vaccines are to be administered for immunizing against influenza and pneumococcal infections.

7. Oil-in-water emulsion for the use according to one of Claims 1 to 6, where the second vaccine is adjuvanted with an aluminium compound.

8. Oil-in-water emulsion for the use according to one of Claims 1-7, where the second vaccine is a nucleic acid vaccine.

9. Oil-in-water emulsion for the use according to one of Claims 1-7, where the second vaccine is a whole-cell vaccine.

10. Oil-in-water emulsion for the use according to one of Claims 1-7, where the second vaccine is a protein subunit vaccine.

11. Oil-in-water emulsion for the use according to one of Claims 1-7, where the second vaccine is a polysaccharide vaccine.

12. Oil-in-water emulsion for the use according to one of Claims 1-7, where the second vaccine is a polysaccharide conjugate vaccine.

13. Oil-in-water emulsion for the use according to one of Claims 1-12, where the second vaccine is used therapeutically and/or post-exposure.

14. Oil-in-water emulsion for the use according to one of Claims 1-4 and 7-13, where the second vaccine is a rabies vaccine.

15. Oil-in-water emulsion for the use according to Claim 14, where the adjuvant and the vaccine are to be applied for the post-exposure prophylaxis of rabies.

16. Oil-in-water emulsion for the use according to one of Claims 1-6 or 11-13, where the second vaccine is a pneumococcal capsular polysaccharide vaccine or is a pneumococcal polysaccharide conjugate vaccine.

17. Oil-in-water emulsion for the use according to one of Claims 1-4 or 7-13, where the second vaccine is a diphtheria vaccine, a tetanus vaccine, a meningococcal vaccine against serotype A, C or other serotypes, an HIV vaccine, an HBV vaccine, a Helicobacter pylori vaccine, an early summer meningoencephalitis vaccine (ESME) or a typhoid vaccine.

18. Oil-in-water emulsion for the use according to one of Claims 1 to 17, where the adjuvant and the vaccines are to be used for patients with suppressed immunity, diabetics or residents of old people's homes.

## Revendications

1. Émulsion huile dans eau qui comprend 5 % de squalène, 0,5 % de polysorbate 80 et 0,5 % de trioléate de sorbitane dans un tampon citrate aqueux de pH 6,5, destinée à être utilisée en tant qu'adjuvant contralatéral pour un second vaccin, ladite émulsion et le second vaccin devant être appliqués sur des côtés opposés du corps de la personne, et le second vaccin et un premier vaccin devant être appliqués sur des côtés opposés du corps de la personne,
(i) le premier vaccin étant adjuvé par mélange de ladite émulsion avec le premier vaccin à adjuver soit lors de la fabrication du vaccin, soit seulement peu avant l'application, et
(ii) le second vaccin n'étant pas adjuvé par mélange de ladite émulsion avec le second vaccin soit lors de la fabrication du vaccin, soit seulement peu avant l'application.

2. Émulsion huile dans eau destinée à être utilisée selon la revendication 1, pour l'immunisation contre des maladies infectieuses virales, bactériennes ou parasitaires.

3. Émulsion huile dans eau destinée à être utilisée selon la revendication 2, dans laquelle le premier vaccin et le second vaccin sont à appliquer simultanément de manière contralatérale sur des côtés opposés du corps de la personne.

4. Émulsion huile dans eau destinée à être utilisée selon l'une quelconque des revendications 1 à 3, dans laquelle le premier vaccin adjuvé est un vaccin sous-unité protéique contre la grippe.

5. Émulsion huile dans eau destinée à être utilisée selon la revendication 4, dans laquelle le premier vaccin adjuvé est un vaccin sous-unité protéique contre la grippe et le second vaccin non adjuvé est un vaccin polysaccharidique capsulaire contre les pneumocoques ou un vaccin polysaccharidique conjugué contre les pneumocoques.

6. Émulsion huile dans eau destinée à être utilisée selon la revendication 5, dans laquelle les adjuvants et des vaccins pour l'immunisation contre la grippe et les infections pneumococciques sont à appliquer.

7. Émulsion huile dans eau destinée à être utilisée selon l'une quelconque des revendications 1 à 6, dans laquelle le second vaccin est adjuvé avec un composé d'aluminium.

8. Émulsion huile dans eau destinée à être utilisée selon l'une quelconque des revendications 1 à 7, dans laquelle le second vaccin est un vaccin à acide nucléique.

9. Émulsion huile dans eau destinée à être utilisée selon l'une quelconque des revendications 1 à 7, dans laquelle le second vaccin est un vaccin à cellules entières.

10. Émulsion huile dans eau destinée à être utilisée selon l'une quelconque des revendications 1 à 7, dans laquelle le second vaccin est un vaccin sous unité protéique.

11. Émulsion huile dans eau destinée à être utilisée selon l'une quelconque des revendications 1 à 7, dans laquelle le second vaccin est un vaccin polysaccharidique.

12. Émulsion huile dans eau destinée à être utilisée selon l'une quelconque des revendications 1 à 7, dans laquelle le second vaccin est un vaccin polysaccharidique conjugué.

13. Émulsion huile dans eau destinée à être utilisée selon l'une quelconque des revendications 1 à 12, dans laquelle le second vaccin est utilisé de manière thérapeutique et/ou post-expositionnelle.

14. Émulsion huile dans eau destinée à être utilisée selon l'une quelconque des revendications 1 à 4 et 7 à 13, dans laquelle le second vaccin est un vaccin contre la rage.

15. Émulsion huile dans eau destinée à être utilisée selon la revendication 14, dans laquelle les adjuvants et le vaccin pour la prophylaxie post-expositionnelle de la rage doivent être appliqués.

16. Émulsion huile dans eau destinée à être utilisée selon l'une quelconque des revendications 1 à 6 ou 11 à 13, dans laquelle le second vaccin est un vaccin polysaccharidique capsulaire contre les pneumocoques ou un vaccin polysaccharidique conjugué contre les pneumocoques.

17. Émulsion huile dans eau destinée à être utilisée selon l'une quelconque des revendications 1 à 4 ou 7 à 13, dans laquelle le second vaccin est un vaccin contre la diphtérie, un vaccin contre le tétanos, un vaccin contre les méningocoques contre le sérotype A, C ou d'autres sérotypes, un vaccin contre le VIH, un vaccin contre le VHB, un vaccin contre Helicobacter pylori, un vaccin contre la méningo-encéphalite à tiques (FSME) ou un vaccin contre le typhus.

18. Émulsion huile dans eau destinée à être utilisée selon l'une quelconque des revendications 1 à 17, dans laquelle les adjuvants et les vaccins sont à utiliser chez des patients d'immunité affaiblie, des diabétiques ou des habitants de maisons de retraite.
